# EUROPEAN PATENT APPLICATION

(11) **EP 1 593 403 A1**
(43) Date of publication of application: **09.11.2005**
(21) Application number: 04252653.3
(22) Date of filing: 06.05.2004
(51) Int. Cl.: A61M 5/145

(54) **Medical device for quantitatively and chronologically sequenced infusion delivery**

(71) Applicant: Bionica International NV, 1014 BA Amsterdam (NL)
(72) Inventor: Gilbert, Gregory Ford, Orangevale, CA 95652 (US)
(74) Representative: Mercer, Christopher Paul

(57) **Abstract**

The present invention is a medical infusion and aspiration system delivering precisely timed and accurately calculated, adjusted pulsated delivery in high rates of flow delivering an effective profile of pulses tailored to provide momentary spikes of levels of freely available medicines based upon the uptake of the medicine and optimally on real time measurements of the medicine or response of the patient, termed Quantitative Chronological Delivery. The system comprises any pumping mechanism, and optimally a pumping mechanism, and a cassette or cartridge having a reservoir area where the plunger rotates as it advances in reference to the cartridge to provide additional accuracy and overcome the forces of inertia and slip-stick as well as eliminate backlash. Optimally, the systems incorporates an encoded area and an opening for connection to an infusion tube with an in-line sensor area where sampling probes are located. The infusion is adjusted in both amount and duration between pulses to provide quantitatively controlled, chronologically optimized infusion. A motor causes bi-directional pumping to allow for samples to be presented to the sensor area. The system accuracy allows for more concentrated medicines, as a sealed container can eliminate the need for diluting or withdrawing medicine to load a reservoir, and achieves extraordinary accuracy without error correcting software or expensive volumetric measurement and control systems.

## Description

### FIELD OF INVENTION

This invention relates to medical systems, and more specifically, to a medical infusion system which provides precise chronologically tuned and computed pulse delivery of infusions based upon the uptake characteristics of medicines and physiological responses of the patent, which device can also aspirate fluid and perform tests thereon, using a highly accurate, easily operated, disposable and reliable way to deliver any type of liquid or reagent, providing quantitative chronological delivery for treating a multitude of diseases and conditions, including cellular stimulation of metabolic pathways.

### BACKGROUND OF THE INVENTION:

The historical means of delivering medicines have been arbitrarily divided into two types of delivery. The first is pharmaceutical delivery of compounds through chemically based systems using various carriers with specific chemical properties to control the uptake of the active chemicals. The chemical properties react in pre-designed responses to the tissues which are proximate and affected by the active, thus providing the modulated delivery of medicines.

The second means of delivering medicines uses various mechanical and electromechanical systems to modulate the delivery, which unlike chemical delivery systems, generally have limited direct interaction with the patient's tissues and are blind to the physiological responses to the compounds being administered. The current invention provides a unique pulse delivery system which delivers adjusted pulsed amounts of quantitatively precise actives, chronologically corrected to be most effective based upon the uptake characteristics of the medicine as well as the individual patient's real time response to the medicines or compounds being infused, hence the term Quantitative Chronological Medical Infusion Device.

Some medical devices which have attempted to use information from the physical response of the patient to adjust the medical delivery being infused, include a means for checking glucose by probe, and to help control Continuous Subcutanious Insulin Infusion such as the Medtronic wearable insulin pump with glucose reading information, as in Starkweather, US Patent No. 6,694,191 and another truly "closed loop" system such as the Miles Biostator device as in Clemens, US Patent No. 4,055,175. While the approach of these devices may constitute an improvement over devices which have no bio-feedback of the patent's response, they still fail to use uptake information to provide gradients or spikes of medicine, and thus fail to administer medicines in a quantitatively changing chronologically responsive manner as does the present invention.

Quantitative Chronological Delivery, the providing of precise deliveries of medicines in boluses or pulses based upon improved therapeutic rhythms, and the adjusting of the duration between pulses with the amount of each delivery based upon uptake information and/or the responses of individual patients, is a means of operational delivery coined by the inventor, which delivery addresses and uses medicine uptake characteristics and this physiologically variable response by the patient, to cause enhanced or previously unattainable outcomes from the medicine being infused. For example, the use of Quantitatively Chronological Delivery in the preferred embodiment provides body-wide cellular stimulation of metabolic processes which are not stimulated by any other means other than endogenous proper cellular activities.

The causing of additional therapeutic effect with precisely delivered and adjusted boluses tailored to provide rhythms which enhance the efficacy of the medicine, are achieved only with the use of Quantitative Chronological Delivery methods. The purpose of this invention is to provide medical devices which achieve these methodological benefits.

Prior delivery systems also ignore the reality that many compounds have different effects based upon the patient's physical receptiveness and then-existing physical condition which is subject to change on an hourly or minute by minute basis. The activation of cellular receptors is a mechanism of change which has not been recognized and induced by other mechanical systems.
Prior delivery systems use standardized averaged bioavailability uptake information on a broad basis of patients to determine the allowable dosing of medicines based chiefly on the patient's weight, and then adjust the treatment amounts, if at all, depending upon the gross patient outcomes after administration. However these do not use the benefit of gradient changes caused by pulse therapy to increase beneficial responses. These conventional systems are unable to respond to the increase or decrease of receptor activities that can change in mere minutes, a physiological response which can be induce with Quantitative Chronological Delivery. Examples of physiological rhythms which current systems ignore, include almost all endocrine functions from the more obvious circadian rhythm to the very subtle rhythms related to hormonal uptake activities.

The present invention is a means of inducing increased response by pulses using accurate an infusion device with disposable cartridge in its preferred embodiment, providing a system which delivers a unique and improved rhythm of controlled fluids, using boluses or pulses of fluid delivery based upon medicine uptake data and these physiological rhythms as well as, in certain preferred embodiments, ongoing real time biological information on substrate measurement in the patent or the measurement of conditions resulting from the patient's response to the treatment. The biologically superior rhythmic pulse delivery provides a momentary change, that is a gradient which is a superior means designed to induce physiological responses which are not available with more gradual, sustained or unchanging rates of infusion. These momentary changes are the province of Quantitative Chronological Devices, which adjust the medicine delivery in both duration between pulses, and the amount of the bolus, causing differently spaced and differently peaked free levels of the active medicines.

These momentary changes affect therapeutic results irrespective of the absolute value of the medicines, as many tissue responses are induced or repressed by the change in the value of available "free" medicine, not the total amount of free medicine in the patient's system. Thus, the background of medicine and level of medicine free in the patient's body has one affect, but the gradient of bolus (pulse) of the medicine has an entirely different affect on the tissues, which is patterned and controlled by Quantitative Chronological Delivery.

In the field of pumping, there have long existed numerous ways to pump and infuse liquids for medial use, beginning with a simple syringe or pipette and progressing to highly sophisticated electromechanical software correcting systems of gates and valves with many moving parts and checking systems. In addition, not directly related to infusion devices, there exists a large number of blood and tissue testing diagnostic technologies which can detect, identify and quantify the presence and levels of various substrates in plasma and whole blood.

In the present invention, the providing of patterns and adjustment of patterns of treatment can be used alone for certain conditions, or in several preferred embodiments, in conjunction with real time diagnostic testing for improved medical outcomes, and the establishment of interactive delivery modalities which deliver medicines adjusted for individually beneficial levels and timing of the delivery, customized to achieve improved therapeutic responses. The present invention provides a means to deliver this system of timed and accurate pulses to treat a number of different conditions and disease states with different medicines, by providing boluses of medicine based upon rhythms, medicine uptake, and in preferred embodiment, real time sensing of levels, and as to certain conditions, the ingestion of other foods or fluids.

One example of the present invention is the treatment of metabolic syndromes, burn victims and healing disorders through the infusion of intravenous insulin, delivered with the new Quantitative Chronological Delivery of timed bolus doses adjusted to every four to seven minutes, with individually tailored and constantly adjusted amounts of delivery, to cause momentary gradients of circulating insulin levels which result in the expression, repression, activation and inactivation of enzymes and other physiological reactions, and thus result in greater medical efficacy and unique beneficial outcomes. Eight of the disease states which respond quickly to Quantitative Chronological Delivery of insulin are heart and cardiovascular disease, central nervous system disorders, diabetes, retinopathy, psychological disorders, kidney disease, the treating of wounds from injury or surgery, and chronic fatigue syndrome.

First among these diseases which respond to the Quantitative Chronological Delivery of insulin are metabolic disorders, diseases which are not merely diseases of improper blood glucose, but rather diseases of improper body-wide active and resting metabolism. By using Quantitative Chronological Delivery of a series of specially timed boluses of adjusted insulin, while in the presence of glucose, principally from oral ingestion, the resting metabolism of every person, including a diabetic person can be beneficially shifted from elevated lipid and free fatty acid based, to greater carbohydrate based, among other results. The use of Quantitative Chronological Delivery, for metabolic diseases through the subject invention, addresses the core of diabetes and provides an optimized means for correcting and avoiding the secondary complications of diabetes.

In order to demonstrate that some benefit could be achieved with prior methods, the Bionica 110 infusion device was modified under the direction of this inventor to provide a pulsed treatment of insulin which did not calculate changes to the doses or interval for the infusion of insulin. While this prior work demonstrated that the Bionica 110 pump delivery of pulses of insulin, coupled by adjusted oral glucose, could be somewhat effective; significant diagnosis, adjustments, and testing by a health care professionals and intervention was required to obtain some success. Achieving the desired levels and metabolic changes using this method was an inexact science with intermittent results due to the limited approach to adjusting the delivery doses and adjusting the glucose to "cover" the insulin infused. This method of providing and adjusting glucose after a series of pulses of insulin requires a trial and error approach of guessing the effect of an amount of insulin, giving the insulin, and then guessing the amount of glucose needed to "cover" the infused insulin, which is reacting, after the fact, to a change in blood glucose. Information from a standard respiratory quotient measurement machine can be used to determine the effect of the pulses, and to determine whether metabolic changes are taking place.

Prior systems have produced varied and uncertain levels of metabolic and physiologic change, and required significant experience to operate and deliver the treatment in the way it has been delivered. In diabetes, in large part, the difficulties were due to the need to use glucose to manage the treatment. Prior systems provide insulin in a variety of ways, and then provide glucose to cover that insulin. However, as is now proffered by Quantitative Chronological Delivery, it is more effective to adjust the doses and intervals of infusion, and not adjust the glucose to the insulin doses. Less than optimal cellular stimulation occurs by adjusting and providing glucose after receiving insulin as shown by metabolic measurement. Without this invention, inconsistent physiological responses, such as the activation of counter-regulatory systems can cancel the effect of a treatment. The present invention departs from customary treatment of covering insulin with glucose, and by providing Quantitative Chronological stimulation, avoids this basic problem of activating counter-regulatory responses, and avoids inconsistent results while providing significantly better and more uniform outcomes. From the study of prior deliveries, giving glucose to cover insulin is effectively backwards.

By using the present invention with (1) a more accurate deliveries of exacting pulses and (2) in the preferred embodiment a system to adjust the bolus deliveries in amount and timing, Quantitative Chronological Delivery treatments can be practiced with consistently better results, more automation, and without the operator estimating or guessing after the fact, the amount of counter-reagent to be given by mouth or other means to cover the levels of free medicines in the patient. This invention thus provides a basic change from the prior therapy. Prior approaches also did not attempt to read and adjust the amount of reagent during administration. Rather, infusions were given in set amounts, and counter-reagent adjusted to keep the patient from excursions outside of customary levels. Customary ranges of glucose levels in the prior approach ranged from 60 milligrams per deciliter to over 400 milligrams per deciliter of glucose, sometimes in the same sitting. With this invention, the customary patient ranges in blood glucose will be from 70 to 180 milligrams per deciliter, avoiding the extreme swings or excursions in blood glucose. Similar blunting of other physiological responses will be seen in other conditions due to the nature of the pulse delivery, the more accurate administration and the shorter duration between testing. In prior work, the duration between pulses has been kept at a constant 6 minutes between each treatment, and thus, in diabetes, glucose was used to cover set pulses, after finger sticks determined the blood glucose levels, and the amount of glucose was either given or withheld. This prior approach was dictated by the state of technology then existing.

In the present invention, as with optimal insulin delivery in humans, the insulin is now adjusted, not the blood glucose. Insulin is provided in response to the glucose load, not, as used in prior work, glucose adjusted in response to, and to cover, the presence of insulin.

One major advantage of the invention relates to consistent stimulation of tissue without disruptive excursions in blood glucose or other substrates. Under the prior system, patients experienced wide swings of blood glucose, and indeed were kept at an artificially high blood glucose level to mitigate the risk of hypoglycemia. When the patient's response to the set amount of insulin caused quickly lowering glucose levels, the prior system would require that the technician mentally calculate the speed of drop, and give oral or intravenous infusion of high glucose indexed foods. When this happened, not only is the patient not receiving the benefit of the treatment, the patient depleted his or her glucose stores in liver and other tissue, to help cover the dropping circulating glucose, and concominentantly launched counter-regulatory secretions as a defense processes, making the treatment ineffective.

In the metabolic syndrome and diabetes models, in order to get proper stimulation of tissues without adverse reactions from quickly changing glucose, it is necessary to avoid the excursions of blood glucose, and deliver the bolus doses tailored for the physical requirements of that particular moment. In the prior form of treatment, the length of effect of the treatment depended upon the skill of the provider, and would last from almost no time, to less than one week. This is a failure of metabolic changes which are the goal of the therapy, as needed for the treatment of this disease. The new invention avoids these failures to achieve Quantitative Chronological Delivery, and in the case of insulin, does not merely attempt to cover insulin with a post-treatment adjustment of glucose intake. In the case of other medicines, the avoidance of excursions and various counter-regulatory responses likewise allow the medicines to be used by the target tissues and organs.

The present invention avoids harmful excursions of blood glucose or other counter-regulatory responses common to most infusions, and their resulting lack of therapeutic effect by Quantitative Chronological infusions, by providing unique adjusted bolus delivery and by increased accuracy with a better means of adjusting the active, such as insulin, including the use of real time assessment of physiological responses to the infusions. The present invention thus achieves its unique results by incorporation of the more accurate bolus system into the device and the entry of more real time test data to control the infusion by the device.

The indications for use of Quantitative Chronological Delivery are found in specific conditions as observed by the inventor using the Bionica device. All of these methods have used the inventor's Bionica pump in the delivery of the treatment, and attempt to achieve success through the assessment of the health care provider after significant training and trial and error. This required skill caused the inventor to seek a better means for delivery, and resulted in this patent. The present invention removes the trial and error approach, and avoids the need for post-treatment adjustments by having real-time changes in the bolus treatment, entered directly or as a result of various tests which are conducted during the treatment. The examples of treating these diseases including diabetes and related conditions are only a few examples of the need for Quantitative Chronological Delivery, as tissue response to rhythms will be the new standard of delivery.

With Quantitative Chronological Delivery and the optimization of therapy through adjustments of the infusion and customization for the rhythms found in human physiology, a new level of controlling medical infusions is achieved. This interaction between patient and device was previously thought to be too complicated to achieve due to the large variety of physiological responses from most treatments themselves. However, Quantitative Chronological Delivery studies show that adjusted precise bolus treatments can induce additional efficacy from the pattern of accurate delivery. And, with real time test information the patterns of bolus deliveries to have an even greater effect on tissue and the corresponding diseases or conditions.

The subject invention thus addresses the need for medicines which are improved with pulse delivery and real time information on patient reactions to infusion by Quantitative Chronological Deliveries. While some medicines can be administered using this system without any real time tests or monitored levels, relying upon the gradient change effect of the improved accurate bolus delivery provided by the invention system, all current medical infusions can be further optimized with real time tests and adjustments. The invention device also eliminates the difficulty of choosing to either adjust the infusion or providing an ameliorative treatment post infusion. The invention device eliminates the difficulties caused by a counter-regulatory response from the body's own mechanisms.

Another problem with prior intravenous insulin systems is hyperglycemic or hypoglycemic blood conditions, which are addressed by Quantitative Chronological Deliveries. There is a resulting lack of effective treatment when hyperglycemic or hypoglycemic excursions take place, sometimes invoking the response by a patient of "dumping" the liver glycogen stores, coupled with epinephrine release. This problem is sufficiently important that one clinician delivering IV Insulin has claimed that the failure to be properly trained can result in danger to the patient. The present invention is designed to eliminate such potential dangers with an automatic response to real time testing and Quantitative Chronological Delivery, making this invention a significant improvement in safety for intravenous insulin and other medicines equally responsive to Quantitative Chronological Delivery.

The current invention also avoids the effect of not achieving physiological results from the failure to properly estimate the glucose or counter reagent that is needed to cover or control the action of the set amount of insulin or other reagent. The automatic and better results from treatment by this invention, are achieved by using more precise bolus deliveries, known uptake characteristics and existing diagnostic tests as accessed by the reversing mechanism of this invention.

In one embodiment of the current invention, the device provides for measuring levels of substrates on a real time basis. This avoids manual monitoring of blood conditions and reduces both failure to achieve therapeutic change, and the significant assessment work by the health care professional for each patient. The current invention is the result of observing the occasional failures and limitations in achieving therapeutic desires, and applying those lessons to the development of this Quantitative Chronological Delivery system.

The improvement in treatment through Quantitative Chronological Delivery through measured adjusted discrete boluses using highly accurate time adjusted infusions, is unique and has not yet been achieved by any other method. The original Bonica 110 was the first step toward this system, but it failed to achieve uniform and predictable results. The final level of improvement is the current invention, the embodiment of adjusting the medicine instead of covering for its effects, using uptake characteristics and aspirated fluid from the venous access to determine the timing and amount of infusion, (not the amount of remedial medicines to cover the infusion), which are needed to achieve enhanced effect and uniform results.

Quantitative Chronological Delivery through the improved infusion system, and the resulting physiological beneficial changes can be either with or without the oral administration of a substance such as glucose. The preferred embodiment of real time direct testing of the levels of insulin or other medicines provides for the determination of levels of the active ingredient by measurement of its compound characteristics, and the adjustment of the pharmaceutical itself, not a post treatment covering of its effect by dilution or counteractive compound. Another embodiment is the same system using an indirect measurement of the pharmaceutical infused, all of which achieves Quantitative Chronological Delivery. These measurements are made during infusion and will often relate to the half-life or absorption and uptake of the compound being infused. Thus the spikes of infused medicines is calculated by its known half life, and Quantitative Chronological Delivery is performed.

Measurements controlling the infusion timing and amounts can be either by direct measurement of the compound in tissue, or indirectly by the physiological reaction of the patient to the medicines. Infusion of boluses at certain points relating to the half-life or absorption of the infused medicine can achieve enhanced pharmaceutical stimulation, and the present invention, for the first time, gives the practitioner the ability to deliver different tailored profiles using Quantitative Chronological Delivery.

Ultra accurate infusion delivery is beneficial for Quantitative Chronological Delivery due to the diluting effect of the normal movement of blood in veins. In order to deliver the treatment in discrete boluses, and also to track changes in the bioavailability and uptake of the infused medicine, a very accurate infusion must be given, as any volume error in the infusion directly causes errors in the treatment, as well as changes in the gradient slope of each Quantitative Chronological Delivery. In the past, improvements in basic pumping systems have centered on the use of electronic controls to compensate for the mechanical limitations of current pumping systems which generally use non-rigid materials and introduce variations in delivery. Many of these more sophisticated pumping mechanisms have valves and chambers which disturb the reagents normally used in such devices.

The basic problem with accuracy has been that in medical applications, there have been only a few instances where bolus infusion has been sought, and in those instances, the conventional pumping systems would merely rely upon starting and stopping the pump to achieve a bolus regimen. Furthermore, the approach to achieving accuracy in pumping has historically been to slow the delivery so that a more precise metering could take place, which is, of course, not desired in Quantitative Chronological Delivery. Most prior products only offer pump accuracy specifications of plus-or-minus 2 to 5 percent, over the entire reservoir, not for each bolus, thereby making individual deliveries much less accurate. Thus, since there has been no apparent need for a device which could both be accurate, and still pump at a relatively high rate of flow, the field of pumping has not included a pump which can be both accurate in a pulsatile delivery, and have high rates of flow. The need for low pressures in current systems limit the ability to modify and use these systems. The use of higher volumes in delivering medicines are often not medically indicated and many of the current systems have resorted to averaging out individual errors to get a reportable accuracy level which is tolerable but not never optimal. In fact, reporting and delivering accuracy over the entire reservoir does not deliver what Quantitative Chronological Delivery requires, and such reporting may mislead the users to believe that individual accuracy of each aliquiot exists in conventional systems, which is not in fact observed by the inventor. The current invention meets these Quantitative Chronological Delivery needs.

In addition, many of the problems associated with mistakes in the delivery of medicines into patients have resulted from errors in the concentrations of the active reagent. The current invention meets the need of providing a means to automatically avoid errors in both concentration and in reagent through medicine identification and inclusion of profile in the bolus system of delivery.

Most medicines have a relationship to the weight of the patient, such that their drug labels give levels of administration which are optimal, and levels which should not be exceeded in the ordinary practice of medicine. The current invention meets the need of using the uptake information and/or automatically checking the type and relative concentration of the reagent to the weight of the patient as entered into the device, and thereby affords additional safety and protection, by requiring the care giver to take extra steps to enter a delivery profile, and by alerting the care giver that such level is outside the suggested levels of administration, warning the care giver of exceeding the allowed levels as attempted to be entered into the programming of the device.

Most medicines are diluted for the simple reason that an intravenous infusion often is "flowing" and diluted with saline, dextrose or other infusion of medicines. Many devices piggy-back the existing flow with additional actives. The current invention, by more accurate delivery can either add to an existing sustained flow access, downstream of the diluted mixture, or deliver a precise amount of reagent to the dilutant mixture.

Most medicines must be withdrawn from a container, and put into the system for administration. The current invention allows for a disposable cassette, ("cartridge "), which is inexpensive and can be used in glass lined or plastic configurations, as both the pumping cartridge, and the shipping and storage cartridge for the agent since no dilution is required. This disposable container also can eliminate the need for withdrawing the medicine with a needle and then infusing it into a bag or other cartridge. It further avoids loss of reagent in the priming of the infusion device.

Most medicines have proteins or other complex molecules which are relatively easily damaged with any type of gate, valve or force which causes shearing upon the opening and closing of the mechanism used to stop the flow. These proteins have the ability to aggregate and become ineffective, thereby giving to the patient a medicine which has changed in its effective concentrations. Shear and aggregation can also occur with having flows in areas which have a high pressure The current invention avoids the problem of shear, and other medicine degrading pressure problems by having no gate or valve, and by having no high pressure closed areas in the travel of the fluid.

Many medicines are delivered in a relatively inaccurate concentration, due to the forces of ionization and collection of medicines on the surfaces of the bag or container being used as a reservoir to store and deliver the medicine. The medicine can collect on the sides of the container, and only delivered in a relatively unknown and short period of time. The current invention avoids this problem by allowing a very accurate delivery and by avoiding a diluted environment.

Many devices vary their delivery according to the viscosity of the fluid being distributed. The current invention does not vary its delivery profile by the thickness of the reagent. Indeed, fluids with very thick and viscous properties are able to be delivered without loss of accuracy.

Many pumping devices which use syringes have no ability to overcome the natural slip-stick or chatter associated with the storage of energy in the elastic and pliable surfaces and structures, allowing for the syringe moving face ("Plunger") to move forward in irregular motions. Hysteresis and the natural tendency of Plungers not to move until a force overcomes the inertia and sticking forces cause deliveries by most syringe pumps to be sporadically subject to differing levels of sticking (sticktion). When these devices overcome this inertia and hysteresis, they tend to overrun and deliver at different speeds. The current invention avoids slip-stick, chatter, overruns and the problem of hysteresis by breaking the seating forces in a lateral motion. This allows for extraordinary accuracy while using common plastics. Accuracy of plus or minus one percent of one microliter over a ten milliliter cassette are easily achieved without error correcting software or expensive volumetric measurement and control systems.

The current invention has only one moving part in relation to the delivery mechanisms. Simplicity allows for more accuracy and lower costs. It also allows for a single handed adoption of the Cassette to the Pumping Device, freeing the other hand and avoiding accidental sticking with "sharps" such as needles which are contaminated with blood or other materials.

For higher pressure applications, the same system can be use in steel or ceramics. Certain deliveries via long catheters can benefit from a stable and accurate system which is not subject to the errors of conventional pumps, and overcome higher pressures within a given area.

The reservoir head can be made to fit the plunger head so that there is very little dead space and thus very little loss of reagent in the final stroke or end of treatment. This is particularly helpful when the cartridge is also used as the storage and shipping container.

Because the device avoids slip-stick, chatter and the forces of hysteresis, and has no gates or valves, it is designed to also be used in a bi-directional application, such as one of the preferred embodiments herein, where the precise amount being withdrawn can be distributed, equally or in successive steps of precise delivery, or the precise amount withdrawn re-inserted into the patient to the "zero" point. The current invention uses the bi-directional accuracy to allow the device to be fitted with any number of probes in line which provide access to measure the properties of a sample, such as blood, and then re-infuse that blood sample back into the patient after it has been tested, or if desired, by second flow direction, deposit that blood into a separate container or depository.

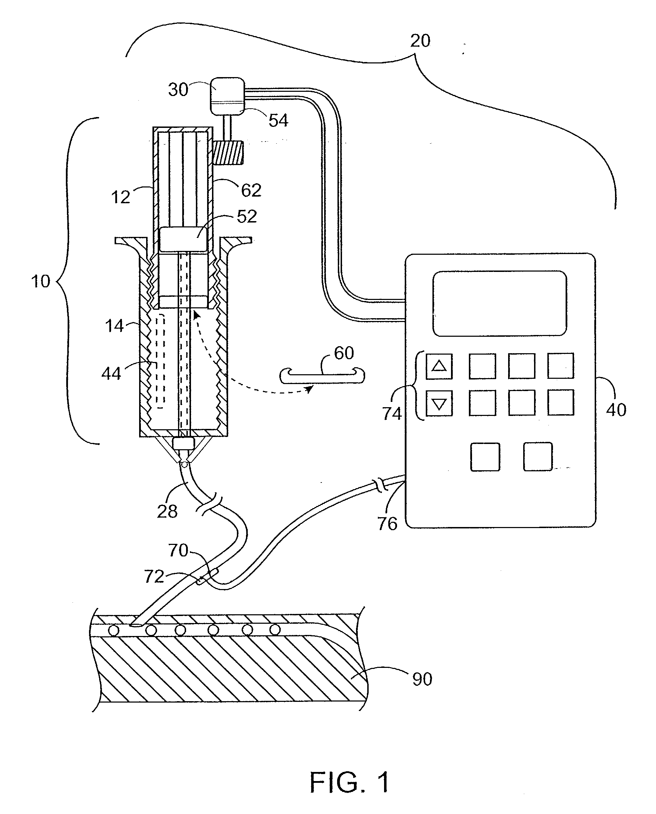

The pumping mechanism can be actuated by any system which rotably moves the plunger or housing, depending upon the configuration. Figures 1, 2 and 3 show a number of different ways to provide this two-axis motion in relationship to the Plunger and Housing. The current invention allows for direct drive, stepper motor, or even spring motor to deliver the amount desired. The "motor" can be even a coordinated hand-eye movement or movement to a series of "click" points.

The pumping mechanism can have a large diameter in relationship to the dept the plunger travels, or a very small diameter and long plunger travel, depending upon the flow characteristics desired for the application. In very viscous fluids, a different diameter would be helpful for both storage and delivery reasons.

The reservoir can be pre-filled, thereby enabling the seller to market pre-filled reagent cartridges. Also, expensive residues (left-overs) are not discarded and lost from the residue in the transportation bottle as pre-filling allows for no waste. The cassette can be removed and re-inserted to store the unused reagent for an appropriate period of time in the Cassette.

The design of the motor and assembly allows the pump to be put above, at, or below the heart level, with no resulting change in the delivery profile. This allows the pump to be worn or enclosed in several different tamper-proof or patient access limiting configurations.

The cartridge when engaged in the delivery device, locks by the rotational providing threads and this locking of the meshed threads makes an accidental infusion by dropping or pressing on the plunger virtually impossible. The cartridge will not siphon out of the pump, or accidentally deliver fluid when dropped or pushed against.

Optical reading of a bar code, or other reading of an area ("Encoded Area") on the cartridge will allow the device to know the limitations on delivery, and mistakes can be reduced as the maximum allowable dose or delivery can be set by each cartridge when filled. When the cartridge is placed in the device, the rotational action causes the Encoded Area to be well aligned and easily read with the uniform motion of screwing the cartridge into place. The rotation, pre-determined position of the Encoded area, and the ease of programming a unique character to each cartridge allows the reagent to be mistake limiting, and since the preferred embodiment system requires a weight to be entered for each patient, the incidence of errors is greatly reduced. Various methods of storing and retrieving data from the Encoded Area are optionally included to provide a system of mistake limitations.

Since the cartridge is also the pumping system, each time the cartridge is used, it is replaced, and the entire wearing aspects of the pumping system are replaced, thereby causing the product life cycles to be much greater. The entire fluid handling system is replaced with each use, and sterilizing and cleaning of parts is eliminated.

All of the foregoing attributes of the invention are beneficial to the proper providing of Quantitative Chronological Delivery, and the providing of the preferred embodiment includes all of the above attributes.

### SUMMARY OF INVENTION:

The object of the invention is to provide Quantitative Chronological Delivery and accurate pulse deliveries using a cassette cartridge pumping and aspirating device (herein "Cassette") which meets the needs as stated above, and which provides a highly accurate, adjusting system for treatment with few moving parts, easily operated, disposable and reliable way to deliver any type of liquid or reagent, primarily for a medial device, but also for other pumping uses. The pumping cartridge, when placed in the housing, causes the piston Plunger to move both forward and aft to infuse or aspirate, as well as rotate within the Cassette to break the forces of inertia and slip-stick as well as eliminate backlash. The result is a Quantitative Chronological Delivery which induces therapeutic effects such as those described herein.

It is another object of the invention to provide an electromechanical as well as only mechanical means to drive and control the pumping device (herein "Pumping Device") for the rotation of the moving parts of the cassette cartridge so as to cause the pumping and aspirating device to either withdraw or pump fluids in response to the plunger of the device moving both in a lateral rotation, and a forward and aft plunging motion, all within in the cylinder of the pumping device containing the reservoir

It is another object of the invention to provide an optical or electromagnetic strip or Encoded Area which, when the cartridge is filled, is encoded with information on the contents and uses of the reagent, and which encoded area, such as a bar code reader, comes into close proximity with a reading system on the electromechanical device which then, as the cartridge is loaded or is first used, causes the medical device to store and use the encoded information in its operations, including a means to limit the profile of the infusion allowed without further intentional override of the profile.

It is another object of the invention to provide a sensor area in the connective lines to the patient so as to determine the chemical components and levels of desired substrates in the aspirated fluids, and use that information to control or limit the infusion profile.

It is another object of the invention to provide more than a single cartridge pump so as to allow the mixing of fluids and the aspiration of fluids in a predetermined pattern and concentration.

It is another object of the invention to provide a cap and container top for the cartridge which allows the cartridge to act as the storage vessel for the reagent, and thereby avoid additional steps of filling, mixing, measuring or wasting reagent in the handling of the fluid.

### DESCRIPTION OF THE INVENTION:

The pumping and aspirating device as seen in Figure 1, is an embodiment of the invention delivering a Quantitative Chronological Delivery, including separate claims to be made, with all features being separate and not necessarily included. The Cassette device with a Plunger, a cylinder area where the reagent is filled, a neck opening in the Plunger for the connection of the cartridge to a tube which travels to where the infusion takes place, and the in-line area where probes for sampling are located to provide input to the pumping device are some of the aspects of the invention which provide the unique delivery modalities.

There are several means to rotate the plunger in relation to the wall of the Cassette. The threaded portion of the cassette requires that the plunger is caused to be rotationally turned as the plunger both infuses and aspirates liquid. An Encoded Area for the placing of encoded information by bar code, magnetic strip, or other encoding system can be located in the immediate area of the surface which is first to be fitted into the cassette holder (the "Encoded Area"). A lip cap is provided to cover to protect the Cassette from contamination or damage to the plunger, and manually removed when inserting the Cassette into the Housing. Openings along the interior sides of the Housing device allow for normally trapped air to be exhausted as the plunger either advances to infuse, or retards to aspirate ("Air space and Inspection Window").

The incline planes forming two meshing screw surfaces are positioned so as to allow the plunger to begin turning as it is first attached, and after it is attached to the cassette Housing. The plunger can extend beyond the line of the Housing for purposes of easy snap-in connection, and alignment of the Cassette. The number of turns per meter or inch are adjusted to provide the desired rate of flow in both directions. The diameter of the cassette and its separate housing are adjusted to provide different flow rates, and to adjust for any necessary fluid dynamics which might be necessary to pump highly viscus fluids, or pump fluids at high rates.

The Housing can either turn or be affixed to the "Pumping Device" with gearing to link the Plunger, Cassette, or Housing to the Motor. The motor can be either electromechanical or a manual wind up, spring or band action motor, adjusted by a mechanical timer for the delivery profile. The motor can also be the manual turning by a hand.

A second cassette and housing can be coupled and driven either independently or in mechanical linkage with a cassette housing so as to have as many infusion profiles, either in succession or concurrently as is desired for the given flow profiles and applications.

On one embodiment, a motor with either electromechanical or mechanical operation such as a spring in mechanical operation, is attached to the gear or drive which in turn causes a rotation of the plunger and both lateral and axial movement of the plunger. The motor is controlled by predetermined settings related to the Quantitative Chronological profile which uses medical uptake information to determine the pulses best suited for the patient, and which may also be controlled by the desired sensor readings, which can be inputted by the operator to cause the pumping and aspiration actions to take place as desired. In the case of a mechanical motor, the settings can be made by a clock mechanism, with the number of turns and speed of the motor determined by the movement limitations of the terming. The uptake information for this embodiment is used to determine the pulse amount and duration between pluses by taking the clearing rate of uptake for that medication and fixing a time which is before the full clearing time such that the amount of the pulse and the time between pulses is adjusted to achieve maximum change in the momentary presentation to tissue of the medicine, without a significant increase in resulting physiological response.

The pumping device can have one, two, three or more sources of input, beginning with the input system to drive the device ("Input") the input of sensors for in-line for measurement of substrates ("In-line Sensor Probes") an in-line occlusion pressure sensing system from the line pressure, or an occlusion sensor from back pressure on the motor, ("Occlusion") and/or input from the reading of the encoded area. Other traditional pump features are intended to be incorporated into the final Pumping Device System.

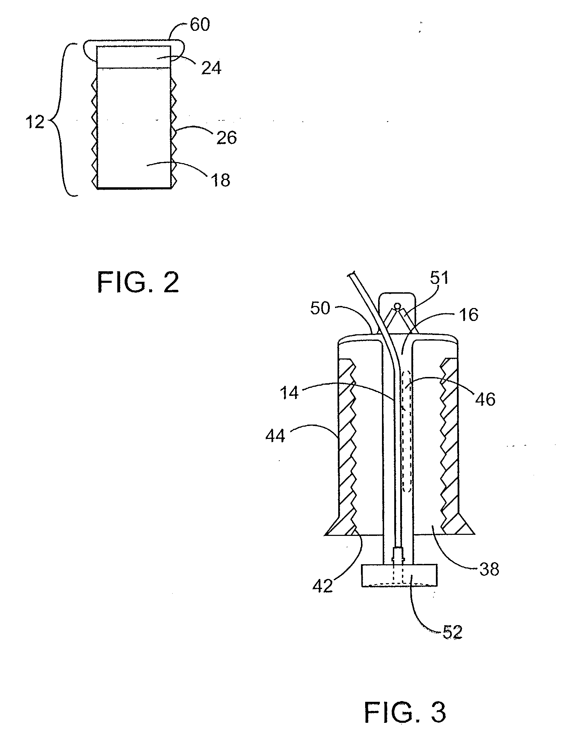

Figure 2 shows the Cassette with only one opening, where the Plunger also provides the locking connection system to the infusion tube. A standard lure lock attachment, or other quick connection system would be included in the plunger as a single piece. A "Protective Cap" is shown removed from the single piece cassette. Splines or grooves on the side of the cassette mesh with a gearing mechanism driven by the Motor, all of which are attached to the "Pumping Device" system. The motor rotates the cassette by attachment to splines on the side, with the Housing fixed to the Pumping Device. The plunger then rotates in reference to the cassette, due to the locking of the plunger to the Housing by the stanchion.

Figure 3 shows a direct screwing interface to the side of the Cassette to accomplish the rotational and axial movement required to provide the delivery profile, as well as a clamshell opening system for easy removal of the cassette and an internal stanchion to hold the Plunger which automatically causes the Plunger to turn in reference to the cassette as the motor advances the cassette upwards and downwards in order to infuse and aspirate.

Also shown is a "Rack" threaded surface which allows the motor, which can be placed adjacent to the Rack, to turn the Housing, while the Plunger remains stationary in relation to the Motor and Rack, thereby causing the Plunger to move rotationally in reference to the Cassette. The Plunger stanchion can swing away for easy snap-in and snap-out action.

In the preferred embodiment, the Rotational Velocity exceeds the Axial Velocity, although with sufficient diameter the difference in Rotation travel to Axial travel could be adjusted for the flow characteristics of the fluid to be infused and aspirated.

All Housings except as to one version of the Rack as shown in Figure 3, can be made in a clamshell or disassembled manner for easy withdrawal.

The delivery profile is designed to allow increasing or decreasing free levels of medicines by infusion in pulses in accordance with the clearing of the medicine by the patient's body, so as to allow pulse delivery and an increasing, stable, or decreasing free level of medicine, as determined by the half-life of the medicine or the Sensors.

The purpose of the preferred embodiment is to provide a system of Quantitative Chronological Delivery a term coined by the Inventor. The benefit to having individually delivered pulses of differently spaced and with different amounts of pulse, to respond to changes in the body, such as the amount of glucose in the system. Part of the invention claimed is the use of the device in a sequence of infusions which, while in the aggregate the amount of medicine used is less, but by virtue of the pulses, provides bioavailability in dynamic stimulation and accomplishes additional medical results.

### SUMMARY

Accordingly, the present invention is a medical infusion and aspiration system delivering Quantitative Chronological infusions of accurate pulsatile delivery at high rates of flow. Another important characteristic of the invention is that the device provides a means to automatically avoid errors in concentration, reagent and medicine type, and avoids the problems of shear and other medicine degrading pressure problems. The system also avoids the slip-stick, chatter, overruns, and the problem of hysteresis by breaking the seating forces between Plunger and Cassette wall in a lateral motion that does not vary the delivery profile, and overcomes any limitations in the viscosity of the reagent. The system also eliminates the need to dilute reagents to provide additional control for the limitations of accuracy in other systems. Other important characteristics of the invention include disposability, inexpensive cost and use by the manufacturer in glass lined or plastic, for the Cassette to act as both the pumping cartridge and the shipping and storage cartridge thus avoiding loss of reagent in the priming of an infusion device. The current invention also eliminates the need for withdrawing the medicine with a needle and achieves extraordinary accuracy without error correcting software or expensive volumetric measurement and control systems.

The current invention consists of a cassette cartridge pumping and aspirating system. The cassette cartridge contains a plunger, a reservoir area where the reagent is filled, a neck opening for the connection of the plunger or cartridge to a tube which travels to where the infusion takes place, and an in-line sensor area where probes for sampling are located. The in-line area probes are used to provide input to the pumping device. The current invention has only one moving part in relation to the delivery mechanism. Simplicity allows for more accuracy and lower costs. It also allows for a single handed adaptation of the cassette to the pumping device, freeing the other hand and avoiding accidental sticking with "sharps" such as needles which are contaminated with blood or other materials.

In a preferred embodiment, the cartridge is cylindrical in shape and has a reservoir area, and an encoded area. The cartridge may be made of glass, plastic steel or ceramics. It is preferable that part of the outer surface of the cartridge be threaded, and part of the outer surface be grooved to accept and mate with a turning gear. The reservoir area is preferably used for containing a reagent and may be pre-filled, thereby enabling the seller to market pre-filled reagent cartridges. The preferred embodiment eliminates expensive residue that is thrown away with the transportation bottle, as prefilling allows for no waste. Pumps which may be re-inserted can store the unused reagent for an appropriate period of time in the cassette.

The neck opening, when not contained in the Plunger, is preferably located at the bottom surface of the Cassette cartridge and sized to connect an infusion tube to the cartridge. Any conventional tube connection device may be used to connect the infusion tube to the cartridge. The cartridge also has a cap and container top which protects the plunger and its opening, and allows the cartridge to act as the storage vessel for the reagent, and thereby avoid additional steps of filling, mixing, measuring or wasting reagent in the handling of the fluid.

In the preferred embodiment of the invention, an optical or electromagnetic strip is located within an encoded area on the cartridge. When the cartridge is filled, an optical or electromagnetic strip with information on the contents and uses of the reagent is placed in the encoded area. The encoded area is preferably located on the outer surface of the cartridge in the area that is first inserted the housing. When the cartridge is placed in the device, it is preferable that the rotational action causes the encoded area to be well aligned and easily read with the uniform motion of screwing the cartridge into place. The preferred rotation, pre-determined position of the encoded area, and the ease of programming a unique character to each cartridge allows the reagent to be mistake limiting. Furthermore, the preferred embodiment system requires the weight of the patient to be entered into the pumping device for each patient, thus greatly reducing the incidence of errors. Any conventional method of storing and retrieving data from the encoded area are preferably included in the present system to limit the incidence of errors. It is preferable that the encoded area comes into close proximity with a reading system as the cartridge is loaded or is first used. The reading system may be any commercially available system capable of reading the encoded area. A medical device stores and uses the encoded information in its operations, including a means to limit the profile of the infusion allowed without further intentional override of the profile.

In the preferred embodiment, the housing consists of a cylindrical tube that is sealed at the upper end, made from plastic and opens at the bottom end to allow cartridge removal. The inner surface of the housing is preferably threaded and sized to receive the cartridge. A plunger is preferably connected to the sealed end and is suspended in alignment with the central axis of the cylindrical tube by a stanchion. In the preferred embodiment, there is a plurality of openings cut through the housing to allow for normally trapped air to be exhausted as the plunger either advances or retards. The plurality of openings also creates an inspection window within the housing allowing visual access and access to the optical or electromagnetic strip within the encoded area. A lip at the bottom of the housing provides for a manually removable cover used to protect the cartridge from contamination or damage to the plunger. When the cartridge is engaged in the housing, the cartridge is locked into place by the rotational engagement of the threads. The locking of the meshed threads makes an accidental infusion by dropping or pressing on the plunger virtually impossible. The cartridge will not siphon out of the pump, or accidentally deliver fluid when dropped or pushed against, which overcomes a major disadvantage to conventional cartridges or syringes.

The preferred plunger is a piston-type plunger and is preferably connected by a stanchion to the sealed end of the housing and is aligned with the central axis of the housing. The plunger head is preferable concaved to trap air and allow for bubble removal, and when an opening is provided on the cartridge, the plunger is sized to fit any reservoir opening, so there is very little dead space thus resulting in very little loss of reagent in the final stroke or at the end of treatment.

In the preferred embodiment, a mechanism is used to rotate the plunger within the cassette. The mechanism comprises a gear linkage to the motor, a motor and a gear connection to the cartridge which acts as the pumping device. The pumping mechanism may be actuated by any motor which rotably moves either the plunger or housing, with the other of the two fixed. The present invention allows for direct drive, stepper motor, spring or band action motor, or hand articulation to deliver the desired plunger rotation. The "motor" may be even a coordinated hand-eye movement or movement to a series of "click" points. In a preferred embodiment, the plunger rotates in relation to the walls of the cartridge.

In the preferred embodiment, the cartridge, when placed in the housing, causes the piston plunger to move both forward and aft to infuse or aspirate, and at the same time rotate the plunger within the cartridge to break the forces of inertia and slip-stick as well as eliminate backlash. Because the device avoids slip-stick, chatter and the forces of hysteresis, and has no gates or valves, it is designed to also be used in a bi-directional application, such as one of the preferred embodiments herein, where the precise amount being withdrawn may be distributed, equally or in successive steps of precise delivery, or the precise amount withdrawn re-inserted into the patient to the "zero" point.

A sensor area located in the infusion tube contains probes designed to determine the chemical components and levels of desired substrates in the aspirated fluids. The determination can be by direct measurement of the medicine, or by measurement of the response of the patient to the medicine. The information obtained by the probes relayed to the pumping device and is used to control or limit the infusion profile, including the changing of both the time between pulses and the amount pulsed.

The bi-directional accuracy of the present invention allows the system to be used with any number of probes. It is preferable that the probes measure the properties of a sample, such as blood, and then allow the present invention to re-infuse that sample back into the patient after it has been tested, or if necessary, by second valve or gate, deposit that blood into a separate container or depository.

The present invention also includes a pumping device to control the cartridge. The pumping device preferably has one, two, three or more sources of input. The preferred pumping device includes, but is not limited to, an input system to drive the device, a sensor input for in-line measurement of substrates, an in-line occlusion pressure sensing system and/or input from the reading of the encoded area, and a means for providing Quantitative Chronological input. The sensor input receives signals from the in-line sensor probes. The in-line occlusion pressure sensing system determines the line pressure or back pressure on the motor. Other traditional pump features are intended to be incorporated into the pumping device. In the preferred embodiment, the Rotational Velocity exceeds the Axial Velocity, although with sufficient diameter the difference in Rotational travel to Axial travel could be adjusted for the flow characteristics of the fluid to be infused and aspirated.

It is preferable that a second cassette and housing may be coupled and driven either independently or in mechanical linkage with a cassette housing so as to have as many infusion profiles, either in succession or concurrently as is desired for the given flow profiles and applications.

Since the cartridge also acts as the pump, each time the cartridge is used, it and preferably the plunger are replaced, and the entire wearing aspects of the pumping system are replaced. The product life cycles are much greater and since the entire fluid handling system is replaced with each use and sterilization and cleaning of parts is eliminated.

The purpose of the present invention is to provide a system of Quantitative Chronological delivery, a term coined by the inventor. The apparent benefit to having individually controlled pulses in duration between pulse, and amount infused, of almost any medicine, as an additional means for stimulating tissue and was deemed by the Inventor to be a valid approach to medical infusion for any and perhaps all forms of infusion therapy. Part of the invention claimed is the use of the device in sequence of infusions which, while in the aggregate the amount medicine used is less, but by virtue of the pulses, accomplishes additional medical results.

### DESCRIPTION OF THE DRAWINGS

A preferred embodiment of the present invention is further described in connection with the accompanying drawings, in which:
FIG. 1 is a perspective view of a preferred embodiment of the medical infusion and aspiration system.
FIG. 2 is a perspective view of a preferred embodiment of the cartridge.
FIG. 3 is a perspective view of a preferred embodiment of the housing and plunger.
FIG. 4 is a perspective view of another preferred embodiment of the medical infusion and aspiration device.
FIG. 5 is a perspective view of a third preferred embodiment of the medical infusion and aspiration device.
FIG. 6 is a perspective view of a fourth preferred embodiment of the medical infusion and aspiration device.
FIG. 7 is a perspective view of a fifth preferred embodiment of the medical infusion and aspiration device.
FIG. 8 is a perspective view of an embodiment of the medical infusion and aspiration system having two cassettes being driven independently.
FIG. 9 is a perspective view of an embodiment of the medical infusion and aspiration system having two cassettes coupled by mechanical linkage.
FIG. 10 is a graph of the resulting infusion profile and free levels of medicine when starting with no or low backgrounds of medicines.
FIG. 11 is a graph of the resulting infusion profile and free levels of medicine when staring with a prior amount of medicine present in the body.

### DETAILED DESCRIPTION OF THE INVENTION

The present invention is a medical infusion and aspiration system capable of accurate pulsatile delivery at high rates of flow adjusting the time between pulses and amount of delivery to provide Quantitative Chronological Delivery.

The present invention provides a means to provide changes in timing and amounts of delivery to provide maximum tissue stimulation while automatically avoiding errors in concentration, reagent and medicine type, and avoiding the problems of shear and other medicine degrading pressure problems. The system also avoids the slip-stick, chatter, overruns, and the problem of hysteresis by breaking the seating forces between the plunger and cartridge wall in a lateral motion that does not vary the delivery profile by the viscosity of the reagent. The system also avoids loss of reagent in the priming of a separate infusion device and eliminates the tendency of reagents to separate when in a diluted environment. The invention is disposable, inexpensive and may be used by the manufacturer in glass lined or plastic, as both the pumping cartridge and the shipping and storage cartridge. The current invention, by acting as the storage container, also eliminates the need for withdrawing the medicine from a storage container with a needle and achieves extraordinary accuracy without error correcting software or expensive volumetric measurement and control systems.

In general, the current invention comprises Quantitative Chronological Infusion cassette cartridge pumping and aspirating device. The cassette cartridge pumping and aspiration system consisting of a cartridge, a housing, a plunger, a reservoir area where the reagent is contained, a neck opening in the plunger or cassette for the connection of the cartridge to a tube which travels to where the infusion takes place, and an in-line area where probes for sampling are located. The in-line area probes are used to provide input to a pumping device. The current invention has only one moving part in relation to the delivery mechanism and this simplicity allows for more accuracy and lower costs. It also allows for a single handed adaptation of the cartridge to the pumping device, freeing the other hand and avoiding accidental sticking with "sharps" such as needles which may be contaminated with blood or other materials.

Referring now to the figures, FIG. 1 is a perspective view of an embodiment of the invention showing a cassette 10, a pumping mechanism 20 and a motor 30. The cassette further comprises a cartridge 12, and a housing 14.

In a preferred embodiment, as best seen in FIGS. 1 and 2, the cartridge 12 is cylindrical in shape and has a reservoir area 18, and encoded area 24. The cartridge 12 is preferably made from glass or plastic. For high-pressure situations, it is preferable that the cartridge 12 be made of steel or ceramics. It is preferable that the outer surface of the cartridge be partially threaded at the top and grooved for the remaining area to the end of the cartridge 26. Any standard or metric thread and groove sizes may be used.

The reservoir area 18 is preferably used for containing a reagent. The reservoir area 18 may be pre-filled, thereby enabling the seller to market pre-filled reagent cartridges which also act as the storage and transportation vehicle. The preferred embodiment eliminates expensive residue that is thrown away with a separate transportation bottle, as pre-filling allows for no waste. The preferred embodiment eliminates dilution requirements due to the accuracy of the pumping means. Cartridges which may be re-inserted can store the unused reagent for an appropriate period of time in the cartridge.

The housing is preferably opened along its center axis to remove cartridges, by means of a hinge 51 which is located off of center to allow the tube to run unobstructed up the stanchion and out of the cassette.

The neck opening 22 is preferably located in the plunger 52, or may be located on the bottom surface of the cartridge 12. The neck is preferably sized to connect an infusion tube 28 to the cartridge 12. Any conventional tube connection device may be used to connect the infusion tube 28 to the cartridge 12. The opposite end of the infusion tube 28 is connected to the sensors and then to a vein in the patient 90.

It is preferable that the cartridge 12 also contains a cap as the container top which allows the cartridge 12 to act as the storage vessel for the reagent, and thereby avoids the additional steps of filling, mixing, diluting, measuring or wasting reagent in the handling of the fluid.

In the preferred embodiment of the invention, an optical or electromagnetic strip is located within an encoded area 24 on the cartridge 12. When the cartridge 12 is filled, an optical or electromagnetic strip with information on the contents and uses of the reagent is placed in the encoded area 24. The encoded area 24 is preferably located on the outer surface of the cartridge 12 in the area that is first inserted the housing 14.

It is preferable that optical reading of a bar code or other reading of the encoded area 24 will minimize dosage mistakes, as each cartridge can set the maximum allowable dose or delivery. When the cartridge: 12 is placed in the system, it is preferable that the rotational action causes the encoded area 24 to be well aligned and easily read with the uniform motion of screwing the cartridge 12 into place. The preferred rotation, pre-determined position of the encoded area 24, and the ease of programming a unique character to each cartridge 12 allows the reagent to be mistake limiting. Furthermore, the preferred embodiment system requires a weight to be entered into a pumping device 40 for each patient, and when computed with the allowable dosing based on weight, greatly reduces the incidence of errors. Any conventional method of storing and retrieving data from the encoded area are preferably included in the present system to limit the incidence of errors.

It is preferable that the encoded area 24 comes into close proximity with a reading system as the cartridge 12 is loaded or is first used. The reading system may be any commercially available system capable of reading the encoded area 24. A medical device stores and uses the encoded information in its operations, including a means to limit the profile of the infusion allowed without further intentional override of the profile.

In the preferred embodiment, the housing 14 consists of a cylindrical tube that is sealed at the upper end, as shown in FIG. 3. The housing 14 is preferably made of plastic, however, any suitable commercially available material may be used. The bottom 38 of the housing is preferably open and the inner surface 42 of the housing is threaded. Any standard or metric thread size may be used. A plunger stanchion 16 is preferably connected to the sealed end 50 and is suspended in alignment with the central axis 36 of housing 14. The plunger stanchion is fixed to the housing and is mated with the plunger 52 when the cartridge 12 is inserted into the housing 14. The plunger 52 is fixed to the stanchion and is not allowed to rotate with the cartridge is turned. The housing 14 is sized to threadedly receive the cartridge 12. In the preferred embodiment, there is a plurality of openings 44 cut through the housing 14 parallel to the central axis 36 of housing 14. These openings 44 allow for normally trapped air to be exhausted as the plunger 16 either advances or retards. The plurality of openings 44 also creates an inspection window 46 within the housing 14. The inspection window 46 also allows access to the optical or electromagnetic strip within the encoded area 24. A lip 48 at the bottom 38 of the housing 14 provides for a manually removable protective cap like that used for the cartridge 60 used to protect the housing and the cartridge from contamination or damage to the plunger 16.

When the cartridge 12 the plunger 52 engages the stanchion 16 to lock it into place and then when the cartridge is then engaged in the housing 14, the cartridge 12 is locked into place by the rotational engagement of the threads 26, 42. The locking of the meshed threads makes an accidental infusion by dropping or pressing on the plunger virtually impossible. The cartridge 12 will not siphon out of the pump, or accidentally deliver fluid when dropped or pushed against.

The preferred plunger 52 is a piston-type plunger and is made from plastic, however, any type of non-reactive material may be used. The plunger 52 is preferably connected to the sealed end 50 of the housing 14 and is aligned with the central axis of the housing. The plunger 52 preferably has a concaved face to allow any air to first fill the neck space and be eliminated when the cartridge 12, is inserted into the housing 14, and is preferable sized to fit within the reservoir area 18, so there is very little dead space thus resulting in very little loss of reagent in the final stroke or at the end of treatment.

The plunger 52 and reservoir area 18 configuration may have a larger diameter in relationship to the depth the plunger travels, or a very small diameter and longer plunger travel, depending upon the flow characteristics desired for the application. In very viscous fluids, a different diameter would be helpful for both storage and delivery reasons.

In the preferred embodiment, a pumping mechanism 20 is used to rotate the cartridge with the stanchion 16 and the plunger 52, fixed to the housing 14. Grooves 62 on the side of the cartridge 12 mesh with the gearing mechanism driven by the motor 30, all of which are attached to the pumping device 40. The motor 30 rotates the cartridge 12 by attachment to the grooves 62 on the side of the cartridge, with the housing 14 fixed in relation to the motor or pumping device.

The pumping mechanism 20 comprises a gear linkage 54, a motor 30 and a pumping device 40. The pumping mechanism 20 may be actuated by any motor which rotably moves the stanchion 16 and plunger 52, or rotates the housing 14. The present invention allows for direct drive, stepper motor, spring or band action motor, or hand articulation to deliver the desired plunger rotation. The "motor" may also be a coordinated hand-eye movement or movement to a series of "click" points. In a preferred embodiment, the stanchion 16 and plunger 52 rotate in relation to the walls of the cassette 12.

In one embodiment, a motor 30 with either electromechanical or mechanical operation causes a rotation of the cartridge 12 with the stanchion 16 and plunger 52 fixed to the housing 14, giving both lateral and axial movement of the stanchion 16 and plunger 52 . The motor 30 is controlled by Quantitative Chronological input to cause the pumping and aspiration actions to take place as desired to achieve the free medicine profile. In the case of a mechanical motor, the settings may be made by a spring-like mechanism, with the number of turns and speed of the mechanism being governed by a simple clock mechanism.

The design of the motor 30 and assembly allow the pump mechanism 20 to be put above, at, or below the heart level, with no resulting change in the delivery profile. This allows the pump mechanism 20 to be worn or enclosed in several different tamper-proof or patient access limiting configurations.

The planes formed by the inner surface 42 of the housing and part of the outer surface 26 of the cartridge are positioned so as to allow the cartridge 12 to begin turning as it is first attached, or after the plunger 52 is attached to the stanchion 16. The stanchion 16 may extend beyond the line of the housing 14 for purposes of easy snap-in connection and alignment of the plunger 52 and also the cartridge 12. The number of turns per meter or inch are adjusted to provide the desired rate of flow in both directions. The diameter of the cartridge and its separate housing are adjusted to provide different flow rates and to adjust for any necessary fluid dynamics which might be necessary to pump highly viscous liquids or pump fluids at high flow rates.

As the cartridge 12 is rotationally turned, the device infuses or aspirates liquid, depending on the rotational direction of rotation. The rotational movement of the present invention allows for bi-directional movement and provides accurate infusion or aspiration.

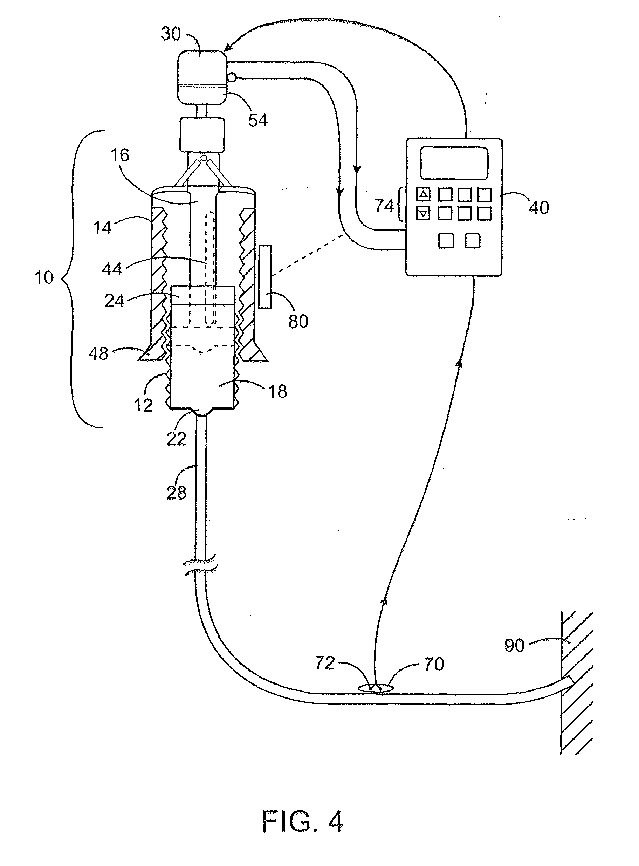

FIG. 4 shows an alternate embodiment of the device. In this embodiment, the cartridge 12 has an opening 22, where the infusion tube 28 is connected to the cartridge. The plunger 16 then rotates, due to the lands and groves meshed between the housing 14 and cartridge 12, causing the infusion and aspiration.

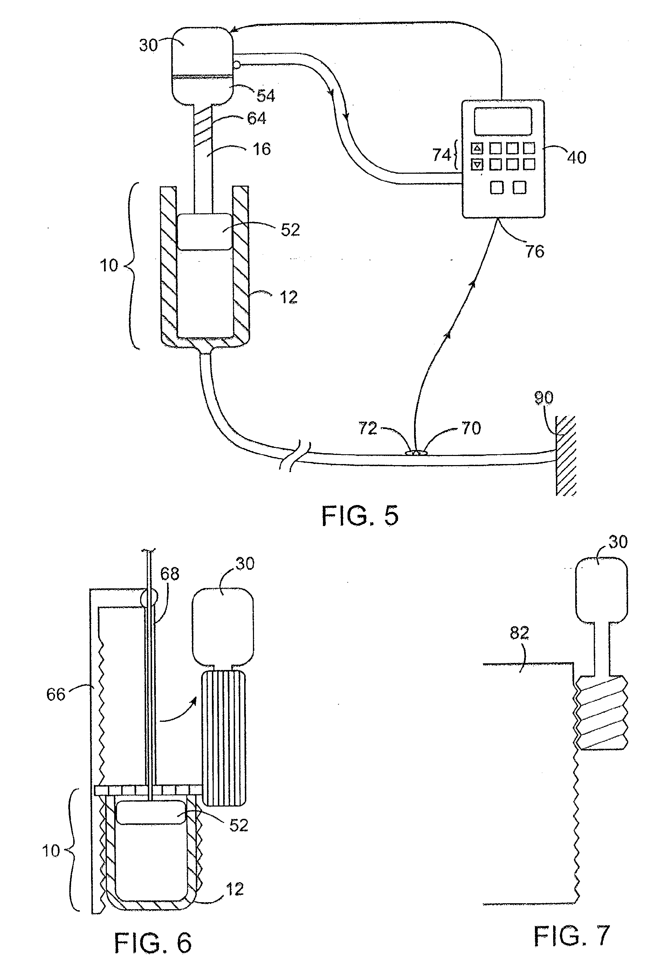

FIG. 5 shows another embodiment of the present invention. In this embodiment, a direct screwing system 64 interface is attached to the side of the cassette 10. The direct screwing system 64 accomplishes the rotational and axial movement of the plunger 52 by dual gearing of the internal rotational drive which automatically causes the plunger 16 to turn as the motor advances the plunger 16 downwards and upwards in order to infuse or aspirate.

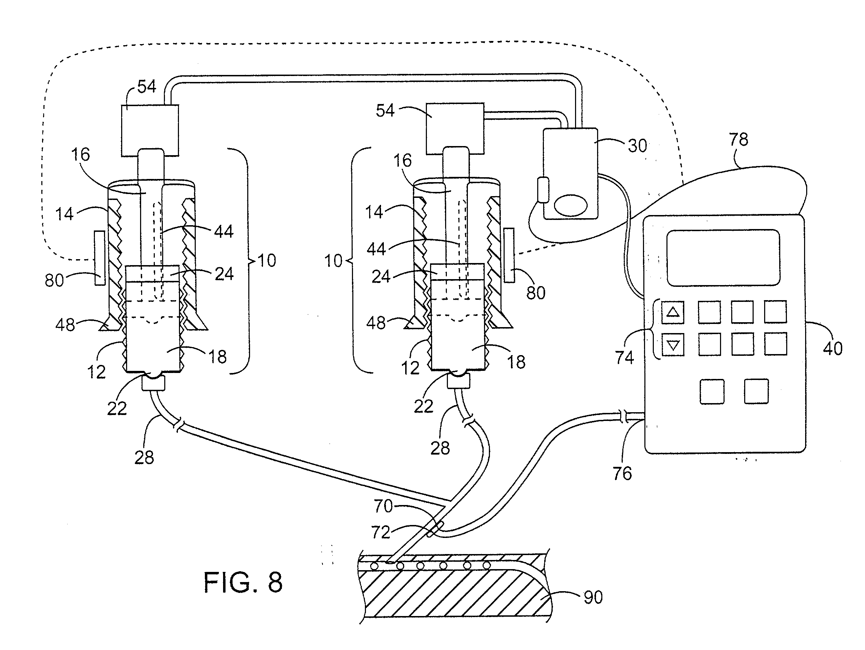

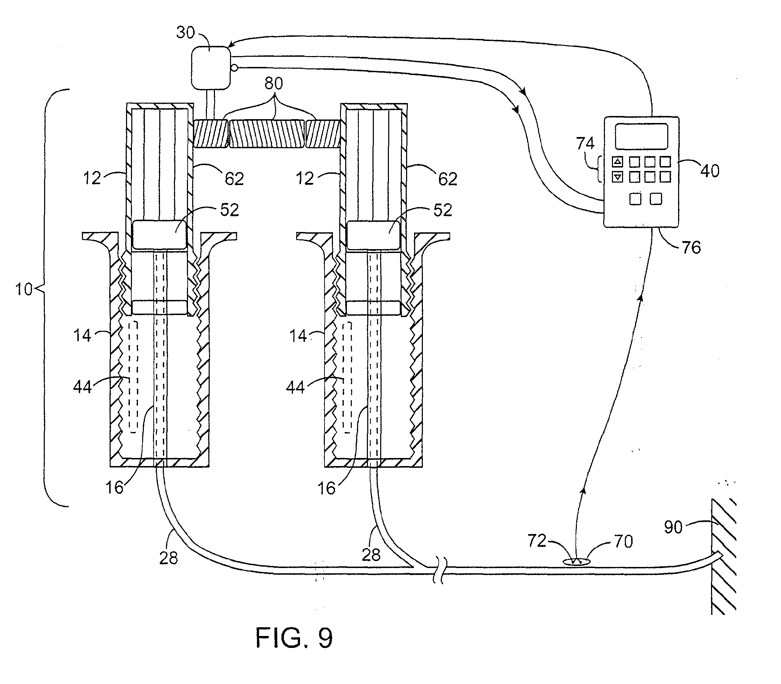

FIG. 6 shows a fourth embodiment of the present invention. FIG. 6 shows a rack 66 threaded surface, which allows the motor 30, when placed adjacent to the rack 66, to turn the housing 14. The plunger I6 remains stationary in relation to the motor 30 and rack 66, thereby causing the plunger 16 to move rotationally in reference to the cassette 10. The plunger sanction 68 may swing away for easy snap-in and snap-out action. FIG. 7 shows a fifth preferred embodiment of the present invention. FIG. 7 shows a side screw 82 configuration for the cassette. FIG. 8 and FIG 9 show multiple cassettes.

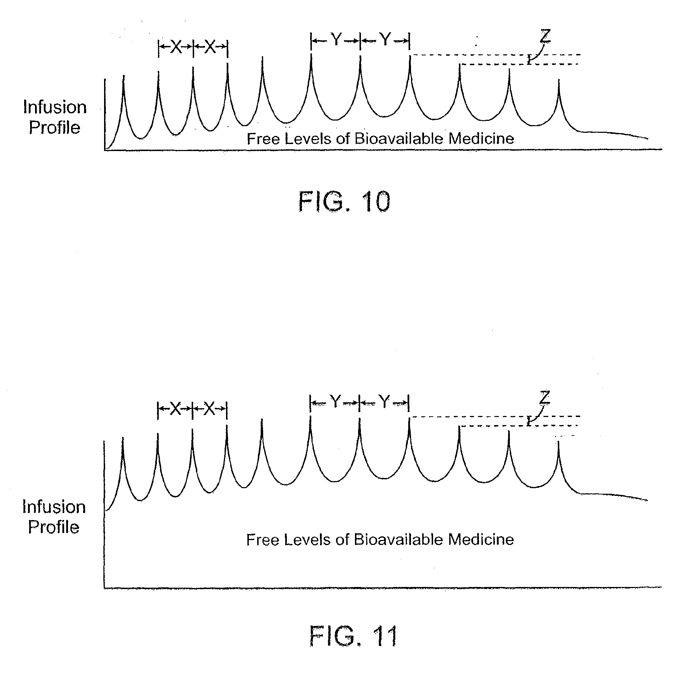

FIG. 10 shows the infusion profile where the infusion begins with no or little background level of bioavailable medicine and the time between infusion X is maintained or changed Y with the concurrent amount of each pulse or bolus changed Z. The profile can be changed to provide increased or decreased baselines of free levels of medicines.

FIG. 11 shows the infusion profile where the infusion begins with some existing background level of bioavailable medicine and the time between infusion X is maintained or changed Y with the concurrent amount of each pulse or bolus changed Z in order to give significant stimulation of the tissue. The profile can be changed to provide increased or decreased baselines of free levels of medicines.

In the preferred embodiment, the cartridge 12, when placed in the housing 14, causes the piston plunger 16 to move both forward and aft to aspirate for testing and then infuse, as well as rotate within the cassette 10 to break the forces of inertia and slip-stick as well as eliminate backlash. The infusion is delivered in pulses where the duration between pulses X is changed Y to increase, maintain or decrease the levels of free medicine which causes beneficial responses by tissues to the medicines. Because the device avoids slip-stick, chatter and the forces of hysteresis, and has no gates or valves, it is designed to also be used in a bi-directional application, such as one of the preferred embodiments herein, where the precise amount being withdrawn may be tested and then re-inserted into the patient to the "zero" point.

In the preferred embodiments shown in FIGS. 1, 4, and 5, a sensor area 70 is located within the infusion tube 28. The sensor area 70 contains probes 72 designed to determine the chemical components and levels of desired substrates in the aspirated fluids. The information obtained by the probes 72 relayed to the pumping device 40 and is used to control or limit the infusion profile as contained in FIGS. 10 and 11. In prototype construction the probes were made of electromagnetic material, however any probe capable of relaying information to the pumping device may be used, including radio frequency, light, infrared waive forms, and chemical testing which is photo sensitive or reactive to the desired substrates.

The bi-directional accuracy of the present invention allows the system to be used with any number of probes. It is preferable that the probes measure the properties of a sample, such as blood, and then allow the present invention to re-infuse that sample back into the patient after it has been tested, or if desired, by second flow direction, deposit that blood into a separate container or depository.

Referring to FIGS. 1, 4 and 5, the present invention also includes a pumping device 40. The pumping device 40 preferably has one, two, three or more sources of input and delivers Quantitative Chronological infusions FIGS. 10 and 11. The preferred pumping device includes, but is not limited to, an input system to drive the device 74, a sensor input for in-line measurement of substrates 76, an in-line occlusion pressure sensing system 78 and/or input from the reading of the encoded area 80. The sensor input 76 receives signals from the in-line sensor probes 72. The in-line occlusion pressure sensing system 78 determines the line pressure or back pressure on the motor. Other traditional pump features are intended to be incorporated into the pumping device 40.

In the preferred embodiment, the Rotational Velocity exceeds the Axial Velocity, although with sufficient diameter the difference in Rotational travel to Axial travel could be adjusted for the flow characteristics of the fluid to be infused and aspirated.

It is preferable that a second cassette and housing may be coupled and driven either independently or in mechanical linkage 80 with a cassette housing so as to have as many infusion profiles, either in succession or concurrently as is desired for the given flow profiles and applications, as shown in FIGS. 8 and 9, with flows as shown in FIGS 10 and 11.

It is a desired effect of the present invention that certain deliveries via long catheters positioned in the patient may benefit from a very stable and accurate system which is not subject to the errors of conventional pumps, even when overcoming higher pressures within a given area.

Since the cartridge and plunger is also the pumping system, each time the cartridge and plunger are used, they are replaced, and the entire wearing aspects of the pumping system are replaced, thereby causing the product life cycles to be much greater. The entire fluid handling system is replaced with each use and sterilization and cleaning of parts is eliminated.

The purpose of the present invention is to provide a system of Quantitative Chronological Delivery, a term coined by the inventor. The apparent benefit to having timed pulses of individually controlled amounts, of almost any medicine, as an additional mode for delivery, was deemed by the Inventor to be a valid approach to medical infusion for any and perhaps all forms of infusion therapy. Part of the invention claimed is the use of the device in sequence of infusions which, while in the aggregate the amount medicine used is less, but by virtue of he pulses, accomplishes additional medical results.

The preferred embodiments described herein are illustrative only, and although the examples given include many specificity's, they are intended as illustrative of only a few possible embodiments of the invention. Other embodiments and modifications will, no doubt, occur to those skilled in the art. The examples given should only be interpreted as illustrations of some of the preferred embodiments of the invention, and the full scope of the invention should be determined by the appended claims and their legal equivalents.

## Claims

**1.** A medical infusion and aspiration system, for accurate Quantitative Chronological Delivery of timed and individually controlled pulses using the known bioavailability uptake data of any infused medicine to deliver the optimal pluses which control or enhance the bioavailability or effectiveness of that medicine, and through pulse delivery, enhance the bioavailability or effectiveness by changing the duration between pulses and amount infused during each pulse irrespective of the existing baseline levels of the medicine, which pulses are delivered by any conventional means of pumping.

**2.** The medical infusion device as in Claim 1 containing:
at least one cassette having a cartridge, a housing and a plunger, the cartridge having a cylindrical shape, an outer cartridge surface, and a reservoir area; the outer cartridge surface having a grooved or threaded surface
an attachment for an infusion tube,
a pumping mechanism having a gear linkage, a motor and a pumping device delivering a pulsatile infusion,
and,
an attachment for an in-line sensor probe.

**2.** The medical infusion and aspiration system of claim 2, wherein the outer cartridge surface is in threaded relationship to the housing inner threaded surface.

**3.** The medical infusion and aspiration system of claim 2, wherein the infusion tube is connectively coupled to the plunger opening.

**4.** The medical infusion and aspiration system of claim 2, wherein the cartridge is configured to receive a cap and a container top.

**5.** The medical infusion and aspiration system of claim 2, wherein the housing has no fewer than one and a plurality of openings parallel to the central axis of the housing; the plurality of openings allows for trapped air to be exhausted and creates an inspection window.

**6.** The medical infusion and aspiration system of claim 2, wherein the bottom surface of the cartridge and housing have lips or ridges to receive a removable cover.

**7.** The medical infusion and aspiration system of claim 2, wherein the encoded area comprises an optical or electromagnetic strip.

**8.** The medical infusion and aspiration system of claim 2, further comprising a mechanism capable of reading the encoded area.

**9.** The medical infusion and aspiration system of claim 2, wherein the stanchion is aligned with the central axis of the housing and configured to capture the plunger fit within the reservoir area.

**10.** The medical infusion and aspiration system of claim 2, wherein the gear linkage connectively couples the motor to the piston or to the cartridge outer surface.

**11.** The medical infusion and aspiration system of claim 10, wherein the motor causes both lateral and axial rotation of the plunger.

**12.** The medical infusion and aspiration system of claim 2, wherein the lateral and axial rotation of the plunger is bi-directional allowing for both infusion and aspiration.

**13.** The medical infusion and aspiration system of claim 2 wherein the in-line sensor probe is located in the infusion tube.

**14.** The medical infusion and aspiration system of claim 13, wherein the in-line sensor probe determines a chemical component or level of substrates in an aspirated fluid.

**15.** The medical infusion and aspiration system of claim 2, wherein the pumping device has at least one source of information input.

**16.** The medical infusion and aspiration system of claim 2, wherein the information provided adjusts the duration between pulses and amount of fluid pulsed to provide Quantitative Chronological Delivery changes to stimulate certain tissues with less active ingredient.

**17.** The medical infusion and aspiration system of claim 2, wherein the at least one cassette comprises at least two cassettes.

**18.** The medical infusion and aspiration system of claim 2, wherein the at least two cassettes are coupled and driven independently or in mechanical linkage.
